(19) **Europäisches Patentamt European Patent Office Office européen des brevets**

(11) **EP 2 663 281 B1**

(12) **EUROPEAN PATENT SPECIFICATION**

(45) Date of publication and mention of the grant of the patent:
**10.08.2016 Bulletin 2016/32**

(51) Int Cl.:
*A61K 9/00* *(2006.01)*    *A61K 31/525* *(2006.01)*

(21) Application number: **11709197.5**

(86) International application number:
**PCT/IT2011/000010**

(22) Date of filing: **12.01.2011**

(87) International publication number:
**WO 2012/095877 (19.07.2012 Gazette 2012/29)**

(54) **CORNEAL DELIVERY OF CROSS-LINKING AGENTS BY IONTOPHORESIS FOR THE TREATMENT OF KERATOCONUS AND RELATED OPHTHALMIC COMPOSITIONS**

KORNEALE FREISETZUNG VON VERNETZUNGSMITTEL MITTELS IONTOPHORESE ZUR BEHANDLUNG VON KERATOKONUS UND ZUGEHÖRIGE OPHTHALMISCHE ZUSAMMENSETZUNGEN

ADMINISTRATION À LA CORNÉE D'AGENTS DE RÉTICULATION PAR IONOPHORÈSE POUR LE TRAITEMENT DE KÉRATOCÔNE ET COMPOSITIONS OPHTALMOLOGIQUES ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**20.11.2013 Bulletin 2013/47**

(73) Proprietor: **Sooft Italia S.p.A.**
**63833 Montegiorgio (FM) (IT)**

(72) Inventors:
• **FOSCHINI, Fulvio**
  **I-00124 Roma (IT)**
• **ROY, Pierre**
  **F-75019 Paris (FR)**
• **STAGNI, Edoardo**
  **I-95127 Catania (IT)**
• **CAVALLO, Giovanni**
  **I-00122 Roma (IT)**
• **LUCIANI, Giulio**
  **I-50012 Bagno a Ripoli (FI) (IT)**

(74) Representative: **Sarpi, Maurizio et al**
**Studio Ferrario srl**
**Via Collina 36**
**00187 Roma (IT)**

(56) References cited:
**WO-A1-2009/158032    WO-A1-2010/023705
WO-A2-2007/025244**

**Description**

The present invention relates to the use of iontophoresis to deliver ophthalmic compositions (in particular collyriums) preferably containing riboflavin, or other cross-linking agents, designed to imbibe the corneal stroma in the practice of the corneal collagen cross-linking (CXL) for the keratoconus treatment, and also relates to the corresponding ophthalmic compositions adapted to be administrated by iontophoresis in the treatment of keratoconus by corneal collagen cross-linking.

According to the present invention it is possible to improve imbibition and penetration of the ophthalmic solution into the corneal stroma also without having to proceed to the removal of the corneal epithelium in the practice of the treatment of keratoconus, or other ectasic corneal disorders and to dramatically reduce the needed treatment time.

Collagen is a fundamental protein of connective tissue in animals, and it is present in the cornea and sclera of the eye. Several eye disorders are related to defect in collagen structure and include keratoconus, keratectasia, progressive, myopia, and possibly glaucoma.

Keratoconus is a degenerative disease of the eye in which structural changes within the cornea cause it to thin and change to a more conical shape than its normal gradual curve. Keratoconus is a genetic disease consisting in a non-inflammatory progressive dystrophy whose evolution may be variable from subject to subject.

Upon onset of this disease, which affects approximately 50 persons in every 100.000 each years, generally young people between 10 and 20 years of age, there appears an irregular curvature that modifies the refractive power of the cornea, producing distorsions of images and a confused close and distant vision. By passing of the time, vision continues regressing irreversibly, with a consequent need for frequent change of spectacles, and for this reason it may at first be mistaken for a myopia associated to astigmatism. After some years the cornea progressively tends to wear out and thin out towards the apex. There then occurs an irregular curvature of the cornea, which loses its spherical shape and assumes the characteristic cone shape (keratoconus). If the disease is neglected, the top can ulcerate with consequent perforation of the cornea; there appear pain, lacrimation and spasm of the eyelids. These changes of the cornea produce an alteration in the disposition of the corneal protein, causing micro-scars that further distort the images and in some cases prevent passage of light, thus giving rise to a troublesome dazzling feeling.

On account of the congenital structural weakness of the corneal stroma due to said disease, after some years the cornea progressively tends to wear out and thin out towards the apex. There then occurs an irregular curvature of the cornea, which loses its spherical shape and assumes the characteristic cone shape (keratoconus).

If the disease is neglected, the top can ulcerate with consequent perforation of the cornea; there appear pain, lacrimation and spasm of the eyelids. These changes of the cornea due to keratoconus produce an alteration in the disposition of the corneal protein, causing micro-scars that further distort the images and in somme cases prevent passage of light, thus giving rise to a troublesome dazzling feeling, above all at times of the day when the sun is low on the horizon (sunrise and sunset).

Current treatments for keratoconus either tend to mask the eye surface irregularity with contact lenses, or attempt to improve the surface contour with intracorneal ring segments, lamellar keratoplasty, or excimer laser surgery. However, the disease is progressive and none of these options obviates the need for eventual corneal transplantation. In fact, when the cornea affected by keratoconus undergoes considerable thinning or if cicatrization occurs following upon lacerations of the corneal surface, surgical transplantation of the cornea (keratoplasty) becomes necessary.

However, in the ophthalmic clinic of the Carl Gustaw Carus University of Dresda in 1997, a new safer and less invasive technique was developed, referred to as "corneal cross-linking" (CXL), which uses in particular riboflavin, activated by a UV laser; from then on this technique has been widely and successfully used in various eye clinics.

Corneal cross-linking is a minimally-invasive method, which uses riboflavin activated by a UV laser (365-370 nm); the method is painless and is carried out in day-hospital. Cross-linking enables reinforcement of the structure of the cornea affected by keratoconus through the interweaving and increase in links (cross-linking) between the fibers of the corneal collagen.

Clinical studies have proved CXL being able to reduce the astigmatism associated to keratoconus as well as to slow down or arrest pathology evolution, thus avoiding the need for transplantation of the cornea. Also other disorders characterized by corneal ecstasia benefit from treatment using the cross-linking method.

Corneal cross-linking is carried out by applying a local corneal anaesthesia for making the abrasion of the corneal epithelium (de-epithelization) having a diameter of 8-9 mm. This is followed by a frequent instillation of a 0.1% riboflavin-based ophthalmic solution, during 15 minutes, followed by irradiation with ultraviolet (UV-A) emitter, during 30 minutes with instillation of riboflavin solution throughout the irradiation operation.

Riboflavin (molecular weight 376, poorly soluble in water), more preferably riboflavin sodium phosphate (molecular weight 456, negatively charged), which is commonly used in corneal cross-linking, is a hydrophilic photosensitizing and photopolymerizing molecule with a poor capacity for diffusing through the epithelium and hence reaching the corneal stroma.

Riboflavin, also known as vitamin $B_2$, is required for a wide variety of cellular processes, it is an easily absorbed micronutrient with a key role in maintaining health in humans and other animals. Riboflavin plays a key role in energy metabolism, and for the metabolism of fats, ketone bodies, carbohydrates, and proteins.

Riboflavin has been employed in several clinical and therapeutic situations. For over 30 years, riboflavin supplements have been used as part of the phototherapy treatment of neonatal jaundice; it has also been used as a muscle pain reliever, and alone or along with beta-blockers, in the prevention of migraine.

In the practice of the corneal collagen cross-linking (CXL) for the keratoconus treatment, riboflavin drops are applied to the patient's corneal surface. Once the riboflavin has penetrated through the cornea, ultraviolet A light therapy is applied. The UV-A riboflavin induced cross-linking of corneal collagen is consisting in the photo-polimerization of stroma collagen fibrils aimed to increase their rigidity and resistance.

The technique has been shown and described in several studies to stabilize keratoconus.

The application of a photosensitizer such as riboflavin-5-phosphate to a tissue, e.g. cornea, skin, tendon, cartilagin, or bone, followed by photoactivation is the object of the invention disclosed in the US patent N. 7,331,350, which can produce a tissue-tissue seal for instance to repair a wound or seal a tissue transplant. Said described method can be applied to different type of surgical procedures such as corneal transplant surgery, cataract surgery, laser surgery, keratoplasty, penetrating keratoplasty, refractive surgery, cornea reshaping, and treatment of corneal laceration is providing a cross-linking tissue method creating tissue seal.

A method for performing oculoplasty for the treatment of corneal dystrophies/keratoconics including applying a riboflavin solution as photosensitizer to a human eye surface and irradiating the treatment region with controlled photoactivating radiation is described in patent application US 2008/0015660.

An ocular solution containing approximatively 0.05-0.25% w/w of riboflavin phosphate and approximatively 20% w/w of dextran for the use in the technique of corneal cross-linking for the treatment of keratoconus is the object of the international patent application WO 2009/001396. The innovative contribution of the dextran to this solution is guaranteeing a good muco-adhesiveness to the ocular surface enabling a better performance of the contact and hence of the impregnation of the corneal stroma by the riboflavin solution.

A very simple formulation relating to a collyrium for the treatment of patients suffering from conical cornea has been recently disclosed by the European patent application EP 2 0253 321. In such formulation, only containing riboflavin-5-phosphate, sodium chloride, benzal chloride and sterile water, the riboflavin photosensitizing substance and the benzal chloride, acting as surface-active agent, assist the penetration of the collyrium in the corneal epithelium; compared to standard collyria four the treatment of conical cornea, the product obtained by this described composition has the advantage of not requiring the removal of the corneal epithelium.

A similar technical solution is obtained through UV-A irradiation of a riboflavin/collagen mixture in the presence of copious oxygen causing rapid cross-linking resulting in adhesion of the mixture in situ effecting its adhesion to underlying ocular structure. Such corneal and scleral tissue seal is disclosed in the International Patent Application WO 2009/073600 in order to obtain a structural augmentation of ocular tissue for better stabilizing progressive corneal diseases.

As demonstrated by the above cited scientific and patent literature, riboflavin (molecular weight 376, poorly soluble in water), and more preferably riboflavin sodium phosphate (molecular weight 456, negatively charged), is the preferred hydrophilic photosensitizing and photopolymerizing molecule mostly used in performing corneal cross-linking; however, it has a poor capacity for diffusing trough the epithelium and hence reaching the corneal stroma.

In order to facilitate the absorption thereof and the complete imbibition of the corneal stroma before starting the irradiation with UV-A, it has been introduced the technique of removing the corneal epithelium (de-epithelization). However, this procedure can create, albeit rarely, complications at a corneal level, pain, in addition to being a method that renders the task of the oculist more difficult.

To overcome said problem, the international patent application PCT/IT2009/000392, and relative priority patent application RM2008A00472, disclose an ophthalmic compositions for corneal cross-linking in the treatment of keratoconus or other corneal ectasic, characterized by the association of riboflavin and specific bio-enhancers in order to try to solve the technical problem of the poor capacity of riboflavin for diffusing through the epithelium and hence reaching the corneal stroma. In fact, by the addition of the disclosed bio-enhancers, the riboflavin based compound facilitates epithelial absorption associated to corneal CXL, avoiding the resort to the removal of the corneal epithelium, enabling a non-invasive corneal elimination or reduction of the anaesthesia and consequent fast healing without pain or possible complication for the patients.

However, despite the important advances in the relevant field of riboflavin solutions, there is still the need of more efficient delivery systems for releasing ophthalmic compositions to imbibe corneal stroma in the practice of corneal cross-linking for the treatment of keratoconus, and of suitable ophthalmic compositions for the treatment of keratoconus specifically formulated to be adapted to the more efficient corneal application as well.

It would hence be desirable to further improve the absorption of riboflavin, to reduce riboflavin administration time, without requiring the removal of the corneal epithelium, hence obtaining a noninvasive corneal cross-linking with elimination or reduction of the anesthesia, which does not need particular post treatment therapy, no edema due to the removal of the epithelium, and consequent fast healing without pain or possible complications for the patient.

Iontophoresis is a noninvasive method which allows the penetration of high concentration of ionized molecules, such as drugs, into living tissue driven by an electric current, in fact, applying a current to an ionizable substance increases its mobility across a biological surface.

Three principle forces govern the flux caused by the current. The primary force is electrochemical revulsion, which propels species of the same charge through tissues. When an electric current passes through an aqueous solution containing electrolytes and a charged material (for example, the active pharmaceutical ingredient), several events occur:

(1) the electrode generates ions,
(2) the newly generated ions approach/collide with like charged particles (typically the drug being delivered), and
(3) the electrorepulsion between the newly generated ions force the dissolved/suspended charged particles into and/or, through the surface adjacent (tissue) to the electrode.

Continuous application of electrical current drives the active pharmaceutical ingredients significantly further into the tissues than is achieved with simple topical administration.

The degree of iontophoresis is proportional to the applied current and the treatment time. It occurs in water-based preparations, where ions can be readily generated by electrodes requiring aqueous media containing electrolytes; so iontophoresis is governed by the extent of water hydrolysis that an applied current can produce. The electrolysis reaction yields either hydroxide OH- (cathodic) or hydronium H30+ (anodic) ions. Some formulations contain buffers, which can mitigate pH shifts caused by these ions. However the presence of certain buffers introduces like charged ions that can compete with the drug product for ions generated electrolytically, which can decrease delivery of the drug product (and increase application time).

The electrical polarity of the drug delivery electrode is dependent on the chemical nature of the drug product, specifically its $pK_a(s)$/isoelectric point and the initial dosing solution pH. It is primarily the electrochemical repulsion between the ions generated via electrolysis and the drug product charge that drives the drug product into tissues. Thus, iontophoresis offers a significant advantage over topical drug application, in that it increases drug absorption. The rate of drug delivery may be adjusted by varying the applied current by the person skilled in the art.

Due to the highly effective administration way of the iontophoretic process, ophthalmologist have long recognized the value of iontophoresis in the delivery of curative molecules to the eye and in the treatment of ocular pathologies, as not only the iontophoretic process permits a more rapid medicine application, but it also allows a more localized and more highly concentrated application of drugs.

Several iontophoretic devices have been developed and improved to be specifically used in eye medical field, and following the technical advances occurred in the last decades in the iontoforesis field, in particular concerning devices and apparatus, currently research and development mainly focus on several optimized formulations suitable for delivery by ocular iontophoresis and methods of use thereof.

Described herein are riboflavin based formulations to be employed in an innovative way for performing CLX deliverable by iontophoresis to treat structural weakness of the corneal stroma, in particular keratoconus, and uses thereof.

Riboflavin sodium phosphate, commonly used in corneal cross-linking, is a low molecular weight, water soluble, negatively charged molecule; such set of features makes it potentially a suitable target for cathodic iontophoresis as shown below.

More in detail, in iontophoresis the three transport mechanisms, chemical, electrical and electroosmotic fluxes are explicited in the Nernst-Planck equation below:

$$Flux_{total} = Flux_{passive} + Flux_{electric} + Flux_{osmotic}$$

According to Prausnitz M.R. and Noonan J.S., "Permeability of Cornea, Sclera, and Conjunctiva: A Literature Analysis for Drug Delivery to the Eye". 1998. Journal of Pharmaceutical Sciences. 87:1479-88, for simplicity it can be assumed that the passive contribution is negligible. The electrorepulsion flow depends on charge (valence), electrical field and concentration, which are proportional to current density and inversely proportional to ions mobility in fluid. In turn, ion mobility depends upon several factors such as concentration, interaction between ionic species themselves and between the ions and the solvent molecule, size of the charged drug molecule, polarity of the solvent,... etc. The electroosmotic flow occurs when an electrical field is applied across a membrane and produces bulk motion of the solvent itself that carries ionic or neutral species with the solvent stream. It is proportional to concentration of both ionic and neutral species of the drug.

The electroosmotic flow is in the direction of the membrane charge's counter-ions. At physiological pH (7,4), skin, like most of the biological membranes including cornea and sclera is negatively charged. Therefore, the electroosmotic flow enhance anodic (+) delivery of positively charged drug while cathodic (-) of negatively charged drug delivery is retarded.

At low pH, over pI, isoelectric value of the cornea and sclera considered to be 4 (see Huang et al, Biophysical journal 1999) and comparable to skin surface's pI values that ranges from 3 to 4, the surface turns positive and electroosmotic flux reverses. That explains the importance of buffering, which besides the fact it protects conjunctival and corneal damage (eye can tolerate a fairly wide pH range and Ophthalmic solutions may range from pH 4.5 - 11.5, but the useful range to prevent corneal damage is 6.5 to 8.5), but it keeps the relative contribution of each flow at a constant level. It also guaranties a stable number of ionic species in the solution if the duration of applied current is kept short.

DESCRIPTION OF THE INVENTION

Described herein are formulations designed to enhance their delivery into and through the eye, adapted to be administered by iontophoresis and relative method thereof. More specifically, the herein described ophthalmic compositions are based on riboflavin or other cross-linking agent having at the same time buffering properties, to be delivered by iontophoresis. In such a way it is possible to improve imbibition and penetration into the corneal stroma without having to proceed to the removal of the corneal epithelium in the practice of the treatment of keratoconus, or other ectasic corneal disorders, by means of corneal cross-linking. Therefore, according to the present invention the ophthalmic solution to be delivered by iontophoresis, preferably has to have an initial pH value in the range comprised between 5-6 in order to act as buffering agent and reach a final pH value not above 9.

The described method for treating keratoconus focuses on developing riboflavin based formulations and use of said formulations to maximize riboflavin delivery through iontophoresis, and patient safety. The application of the described riboflavin based formulations by iontophoresis is novel and suitable for treating corneal ectasia disorders.

Therefore, it is object of the invention to provide a specific product formulation adapted to corneal imbibition associated to CXL to be transferred to the cornea by iontophoresis and to be subsequently irradiated by UV light.

Another object of the invention is to propose an ocular iontophoresis method using a riboflavin solution in a form that is more easily ionizable.

The formulations, which include riboflavin at different concentrations, can be used in presence of different iontophoresis conditions (e.g. current levels and application times). These formulations can, for example, be appropriately buffered to manage initial and terminal pHs, or include other excipients that modulate osmolality. Furthermore, the riboflavin solutions are prepared in a such a way to minimize the presence of competing ions.

This ocular iontophoretic based approach is a novel, non-invasive, and a much more efficient method which can lead to better results than those achieved by classical riboflavin administration ways to introduce riboflavin to the cornea to be treated by CXL. Remarkably, as the administration time is significantly reduced due to increased transfer efficiency, the procedure results much more comfortable for patients.

According to the present invention riboflavin is used in appropriate amounts chosen between 0.001 wt% and 1 wt% with respect to the composition.

In addition, the riboflavin preferably used in the present invention is riboflavin phosphate in appropriate amounts in all the compositions described above; in particular it is preferably present at between 0.05 wt% and 0.4 wt% of the composition of the present invention.

In the preferred embodiment of the invention it has been proven that the riboflavin phosphate solutions used in the achievement of the present invention shows optimal parameters relatively to pH measurements and conducibility making said solutions particularly idoneous to be administered by iontophoresis; in particular, a riboflavin phosphate solution 0.1 wt% of the present invention has a pH value of 5,62 and conducibility of 186.9 $\mu$Siemens/cm at room temperature, a riboflavin phosphate solution 0.2 wt% exhibits pH value of 5,79 and conducibility of 350.0 $\mu$Siemens/cm, while the pH value is 5,93 and the conducibility is 673.2 $\mu$Siemens/cm when the concentration of riboflavin phosphate in the solution raises to 0.4 wt%. So, the pH value of the solution, formulated with an excess of Rib-P-Na, a minimum of sodium phosphate buffer (or other buffering systems), and a pH adjusted below physiologic pH (5-6), during a 1 to 5 min iontophoresis process at an intensity of 1mA, will slowly shift to 8 - 9, which is tolerated by the eye. This feature allows the addition of small amounts of buffer in the solutions which will minimize the competition with Riboflavin being delivered into the eye.

So, in such embodiment, riboflavin monophosphate monosodium salt (Rib-P-Na) used in the formulation will act as a buffer. Upon application of cathodic current, the following reaction will occur at the cathode (water hydrolysis) : $2H_2O + 2e^- \rightarrow 2OH^- + H_2$.

Another embodiment of the invention envisages compositions based on riboflavin specifically formulated to be delivered by iontophoresis and containing enhancers such as bio-enhancers and photo-enhancers.

Bio-enhancers are substances that promote the passage of riboflavin or other photosensitizing and photopolymerizing substances through the corneal epithelium, enabling absorption by the corneal stroma itself, such as for example: EDTA associated to tromethamine, ophtalmologically acceptable EDTA salts associated to tromethamine, polysorbate 80, tromethamine, azone, benzalkonium chloride, cetylpyridinium chloride, cetyltrimethylammonium chloride, lauric acid, menthol, methoxysalicylate, polyoxyethylene, sodium glycholate, sodium glycodeoxycholate, sodium lauryl sulphate, sodium salicylate, sodium taurocholate, sodium taurodeoxycholate.

Photo-enhancers are photosensitive and photopolymerizing substances that can be readily absorbed by the epithelium and that, like riboflavin, can also be activated by light to form corneal cross-linking, such as for example the dyes acridine yellow, quinoline yellow, methylene blue, and erythrosine. In another embodiment of the invention, riboflavin formulated with high molecular weight buffers that will not penetrate cornea and will minimize competition with low molecular weight compound. In this variant, the sodium phosphate monobasic buffer has a relatively low molecular weight compared to riboflavin and will compete during iontophoresis. It could be replaced with higher molecular weight buffers such as HEPES, or PIPES or other ones with similar physical and chemical properties.

The ophthalmic compositions of the present invention can be prepared in the technical form of collyriums and eye-drops, gels, and in any case in all the pharmaceutical technical forms that enable a corneal application followed by iontophoresis according to known techniques; given hereinafter are examples provided by way of illustration, without this implying any limit to the present invention. Other aspects, advantages, and modifications within the scope of the invention will be apparent to those skilled in the art to which the invention pertains.

Formulations are reported below, the dosage of the individual components is expressed in weight percentage.

EXAMPLE 1

| Ingredients | % w/w |
|---|---|
| Riboflavin phosphate | 0,146 g |
| Dextran T500 | 20,00 g |
| $NaH_2PO_4 . 2H_2O$ | 0,225 g |
| $NaH_2PO_4 . 2H_2O$ | 0,950 g |
| NaCl | 0,116 g |
| $H_2O$ | Up to 100 g |

EXAMPLE 2

| Ingredients | % w/w |
|---|---|
| Riboflavin phosphate dehydrated sodium salt | 0.147 g |
| Dextran T500 | 15 g |
| Sodium EDTA | 0.1 g |
| Tromethamine | 0.1 g |
| Dehydrated monobasic sodium phosphate | 0.067 g |
| Dehydrated dibasic sodium phosphate | 0.285 g |
| Distilled water | Up to 100 g |

EXAMPLE 4

| Ingredients | % w/w |
|---|---|
| Riboflavin phosphate dehydrated sodium salt | 0.147 g |
| Dextran T500 | 15 g |
| Quinoline Yellow | 0.050 g |
| Dehydrated monobasic sodium phosphate | 0.067 g |
| Dehydrated dibasic sodium phosphate | 0.285 g |
| Distilled water | Up to 100 g |

EXEMPLE 5

| Ingredients | % w/w |
|---|---|
| Riboflavin phosphate dehydrated sodium salt | 0.147 g |
| Dextran T500 | 15 g |
| Acridine Yellow | 0.050 g |
| Dehydrated monobasic sodium phosphate | 0.067 g |
| Dehydrated dibasic sodium phosphate | 0.285 g |
| Distilled water | Up to 100 g |

EXAMPLE 6

| Ingredients | % w/w |
|---|---|
| Riboflavin phosphate dehydrated sodium salt | 0.147 g |
| Dextran T500 | 15 g |
| Erythrosin B | 0.050 g |
| Dehydrated monobasic sodium phosphate | 0.067 g |
| Dehydrated dibasic sodium phosphate | 0.285 g |
| Distilled water | Up to 100 g |

EXAMPLE 7

| Ingredients | % w/w |
|---|---|
| Riboflavin phosphate dihydrated sodium salt | 0.147 g |
| Dextran T500 | 15 g |
| Methylene blue | 0.050 g |
| Dehydrated monobasic sodium phosphate | 0.067 g |
| Dehydrated dibasic sodium phosphate | 0.285 g |
| Distilled water | Up to 100 g |

EXAMPLE 8

| Ingredients | % w/w |
|---|---|
| Riboflavin phosphate dehydrated sodium salt | 0.147 g |
| Dextran T500 | 15 g |
| Sodium EDTA | 0.1 g |
| Tromethamine | 0.05 g |
| Dehydrated monobasic sodium phosphate | 0.067 g |
| Dehydrated dibasic sodium phosphate | 0.285 g |
| Distilled water | Up to 100 g |

In one embodiment particularly preferred of the present invention, formulations according to examples 1 to 8 provide dehydrated monobasic sodium phosphate and/or dehydrated dibasic sodium phosphate, constituting the buffering system, in variable amounts such to reach the final solution pH value of 5,5, as easily available to the person skilled in the art. It is to be considered also every other buffering systems (such as citrate, acetate, tartaric acid) useful to obtain such a value of pH 5.5-6.

**Claims**

1. An ophthalmic composition for the use in the treatment of keratoconus by corneal iontophoresis **characterized by** the fact to comprise:

   A. cross-linking agents enabling reinforcement of the structure of the cornea affected by keratoconus through the interweaving and increase in links (cross-linking) between the fibers of the corneal collagen and having buffering properties and whose initial pH value is comprised between 5 and 6, wherein the cross-linking agent is riboflavin, or riboflavin phosphate solution, or riboflavin phosphate dehydrated sodium salt, or riboflavin phosphate dehydrated sodium salt.$H_2O$, or riboflavin phosphate dehydrated sodium salt.$2H_2O$, and
   B. a buffering system in variable amounts such to reach the final solution pH value of 5.5, and
   C. substances that promote the passage of riboflavin or other photosensitizing and photopolymerizing substances through the corneal epithelium, enabling absorption by the corneal stroma itself, so called bio-enhancers, and/or
   D. photosensitive and photopolymerizing substances that can be readily absorbed by the epithelium and that, like riboflavin, can also be activated by light to form corneal cross-linking, so called photo-enhancers.

2. An ophthalmic composition for use according to claim 1 wherein the concentration of the riboflavin solution is comprised between 0.1 and 1.0% w/w, and preferably the concentration of the riboflavin solution is comprised between 0.1 and 0.4% w/w.

3. An ophthalmic composition for use according to claim 1 wherein the bio-enhancer is chosen in the list comprising: EDTA associated to tromethamine, ophtalmologically acceptable EDTA salts associated to tromethamine, polysorbate 80, tromethamine, azone, benzalkonium chloride, cetylpyridinium chloride, cetyltrimethylammonium chloride, lauric acid, menthol, methoxysalicylate, polyoxyethylene, sodium glycholate, sodium glycodeoxycholate, sodium lauryl sulphate, sodium salicylate, sodium taurocholate, sodium taurodeoxycholate.

4. An ophthalmic composition for use according to claim 1 wherein the photo-enhancer.is chosen in the list comprising: acridine yellow, quinoline yellow, methylene blue, and erythrosine.

5. An ophthalmic composition for the use in the treatment of keratoconus by corneal iontophoresis according to claim 1 **characterized by** the fact to have the following formulation:

   | | |
   |---|---|
   | Riboflavin phosphate | 0,146 g |
   | Dextran T500 | 20,00 g |
   | $NaH_2PO_4 .2H_2O$ | 0,225 g |
   | $Na_2HPO_4.2H_2O$ | 0, 950 g |
   | NaCl | 0,116 g |
   | $H_2O$ | Up to 100g. |

6. An ophthalmic composition for the use in the treatment of keratoconus by corneal iontophoresis according to claim 1 **characterized by** the fact to have the following formulation:

   | | |
   |---|---|
   | Riboflavin phosphate dehydrated sodium salt | 0,147 g |
   | Dextran T500 | 15,00 g |
   | Sodium EDTA | 0,1 g |
   | Tromethamine | 0,1 g |
   | Dehydrated monobasic sodium phosphate | 0,067 g |
   | Dehydrated dibasic sodium phosphate | 0.285 g |

(continued)

| | |
|---|---|
| Distilled water | Up to 100 g. |

7. An ophthalmic composition for the use in the treatment of keratoconus by corneal iontophoresis according to claim 1 **characterized by** the fact to have the following formulation:

| | |
|---|---|
| Riboflavin phosphate dehydrated sodium salt | 0.147 g |
| Dextran T500 | 15 g |
| Quinoline Yellow | 0.050 g |
| Dehydrated monobasic sodium phosphate | 0.067 g |
| Dehydrated dibasic sodium phosphate | 0.285 g |
| Distilled water | Up to 100 g |

8. An ophthalmic composition for the use in the treatment of keratoconus by corneal iontophoresis according to claim 1 **characterized by** the fact to have the following formulation:

| | |
|---|---|
| Riboflavin phosphate dehydrated sodium salt | 0.147 g |
| Dextran T500 | 15 g |
| Acridine Yellow | 0.050 g |
| Dehydrated monobasic sodium phosphate | 0.067 g |
| Dehydrated dibasic sodium phosphate | 0.285 g |
| Distilled water | Up to 100 g |

9. An ophthalmic composition for the use in the treatment of keratoconus by corneal iontophoresis according to claim 1 **characterized by** the fact to have the following formulation:

| | |
|---|---|
| Riboflavin phosphate dehydrated sodium salt | 0.147 g |
| Dextran T500 | 15 g |
| Erythrosin B | 0.050 g |
| Dehydrated monobasic sodium phosphate | 0.067 g |
| Dehydrated dibasic sodium phosphate | 0.285 g |
| Distilled waster | Up to 100 g |

10. An ophthalmic composition for the use in the treatment of keratoconus by corneal iontophoresis according to claim 1 **characterized by** the fact to have the following formulation:

| | |
|---|---|
| Riboflavin phosphate dehydrated sodium salt | 0.147 g |
| Dextran T500 | 15 g |
| Methylene blue | 0.050 g |
| Dehydrated monobasic sodium phosphate | 0.067 g |
| Dehydrated dibasic sodium phosphate | 0.285 g |
| Distilled water | Up to 100 g |

11. An ophthalmic composition for the use in the treatment of keratoconus by corneal iontophoresis according to claim 1 **characterized by** the fact to have the following formulation:

| | |
|---|---|
| Riboflavin phosphate dihydrated sodium salt | 0.147 g |
| Dextran T500 | 15 g |

EP 2 663 281 B1

(continued)

| | |
|---|---|
| Sodium EDTA | 0.1 g |
| Tromethamine | 0.05 g |
| Dehydrated monobasic sodium phosphate | 0.067 g |
| Dehydrated dibasic sodium phosphate | 0.285 g |
| Distilled water | Up to 100 g |

12. An ophthalmic composition for the use in the treatment of keratoconus by corneal iontophoresis according to any claims from 5 to 11 wherein the dehydrated monobasic sodium phosphate and/or dehydrated dibasic sodium phosphate, constituting the buffering system, are in variable amounts such to reach the final solution pH value of 5,5.

13. An ophthalmic composition for the use in the treatment of keratoconus by corneal iontophoresis according to claim 12 wherein the dehydrated monobasic sodium phosphate and/or dehydrated dibasic sodium phosphate are substituted by any buffering system (such as citrate, acetate, tartaric acid) useful to obtain a pH value of 5.5.

14. An ophthalmic composition according to any claims 1-13 for the use in the treatment of keratoconus **characterized in that** it provides the following steps:

   a. positioning a iontophoretic device on the eye to be treated, the device comprising a reservoir containing said ophthalmic solution;
   b. applying a current to said ophthalmic solution on the eye to be treated;
   c. driving the solution movement by a cathodic current applied for 0.5 to 5 min, at an intensity not higher than 2 mA, and preferably 1 mA.
   d. irradiating, immediately after the end of the current application, of the cornea surface with an UV light for 5 to 30 minutes at a power of 3 to 30 mW/cm$^2$; thereby obtaining the cross-linking of the corneal collagen fibers.

## Patentansprüche

1. Ophthalmische Zusammensetzung zur Anwendung bei der Behandlung von Keratokonus mittels kornealer Iontophorese (Hornhaut-Iontophorese), **dadurch gekennzeichnet, dass** sie nachstehendes umfasst:

   A. Vernetzungsmittel, die eine Verstärkung der Struktur der von Keratokonus betroffenen Kornea durch das Verflechten und die Zunahme von Verknüpfungen (Vernetzung) zwischen den Fasern des kornealen Kollagens ermöglichen und Puffereigenschaften aufweisen und deren Anfangs-pH-Wert den Bereich zwischen 5 und 6 umfasst, wobei das Vernetzungsmittel Riboflavin, oder Riboflavinphosphat-Lösung, oder dehydratisiertes Natriumsalz von Riboflavinphosphat, oder dehydratisiertes Natriumsalz.H$_2$0 von Riboflavinphosphat, oder dehydratisiertes Natriumsalz.2H$_2$0 von Riboflavinphosphat ist, und
   B. ein Puffersystem in variablen Mengen, dergestalt, dass am Ende ein pH-Wert der Lösung von 5,5 erreicht wird, und
   C. Substanzen, die den Durchgang von Riboflavin oder anderen photosensibilisierenden und photopolymerisierenden Substanzen durch das korneale Epithel fördern, eine Absorption durch das korneale Stroma selbst ermöglichen, sogenannte Bio-Verstärker, und/oder
   D. photoempfindliche und photopolymerisierende Substanzen, die ohne weiteres von dem Epithel absorbiert werden können, und die, wie Riboflavin, auch durch Licht aktiviert werden können, um eine korneale Vernetzung zu erzeugen, so genannte Photo-Verstärker.

2. Ophthalmische Zusammensetzung zur Anwendung nach Anspruch 1, wobei die Konzentration der Riboflavinlösung zwischen 0,1 und 1,0 Gew.-% umfasst, und die Konzentration der Riboflavinlösung bevorzugt zwischen 0,1 und 0,4 Gew.-% umfasst.

3. Ophthalmische Zusammensetzung zur Anwendung nach Anspruch 1, wobei der Bio-Verstärker aus der Liste ausgewählt ist, die nachstehendes umfasst: mit Tromethamin assoziierte EDTA, mit Tromethamin assoziierte ophthalmologisch verträgliche EDTA-Salze, Polysorbat 80, Tromethamin, Azone (Laurocapram), Benzalkoniumchlorid, Cetylpyridiniumchlorid, Cetyltrimethylammoniumchlorid, Laurinsäure, Menthol, Methoxysalicylat, Polyoxyethylen, Natriumglycocholat, Natriumglycodeoxycholat, Natriumlaurylsulfat, Natriumsalicylat, Natriumtaurocholat, Natriumtaurodeoxycholat.

4. Ophthalmische Zusammensetzung zur Anwendung nach Anspruch 1, wobei der Photo-Verstärker aus der Liste ausgewählt ist, die nachstehendes umfasst: Acridingelb, Chinolingelb, Methylenblau und Erythrosin.

5. Ophthalmische Zusammensetzung zur Anwendung bei der Behandlung von Keratokonus mittels kornealer Iontophorese nach Anspruch 1, **dadurch gekennzeichnet, dass** sie nachstehende Formulierung aufweist:

| | |
|---|---|
| Riboflavinphosphat | 0,146 g |
| Dextran T500 | 20,00 g |
| $NaH_2PO_4.2H_2O$ | 0,225 g |
| $Na_2HPO_4\text{-}2H_2O$ | 0,950 g |
| NaCl | 0,116 g |
| $H_2O$ | bis zu 100 g. |

6. Ophthalmische Zusammensetzung zur Anwendung bei der Behandlung von Keratokonus mittels kornealer Iontophorese nach Anspruch 1,
**dadurch gekennzeichnet, dass** sie nachstehende Formulierung aufweist:

| | |
|---|---|
| Dehydratisiertes Natriumriboflavinphosphatsalz | 0,147 g |
| Dextran T500 | 15,00 g |
| Natrium-EDTA | 0,1 g |
| Tromethamin | 0,1 g |
| Dehydratisiertes monobasisches Natriumphosphat | 0,067 g |
| Dehydratisiertes zweibasisches Natriumphosphat | 0,285 g |
| Destilliertes Wasser | bis zu 100 g. |

7. Ophthalmische Zusammensetzung zur Anwendung bei der Behandlung von Keratokonus mittels kornealer Iontophorese nach Anspruch 1,
**dadurch gekennzeichnet, dass** sie nachstehende Formulierung aufweist:

| | |
|---|---|
| Dehydratisiertes Natriumriboflavinphosphatsalz | 0,147 g |
| Dextran T500 | 15 g |
| Chinolingelb | 0,050 g |
| Dehydratisiertes monobasisches Natriumphosphat | 0,067 g |
| Dehydratisiertes zweibasisches Natriumphosphat | 0,285 g |
| Destilliertes Wasser | bis zu 100 g |

8. Ophthalmische Zusammensetzung zur Anwendung bei der Behandlung von Keratokonus mittels kornealer Iontophorese nach Anspruch 1,
**dadurch gekennzeichnet, dass** sie nachstehende Formulierung aufweist:

| | |
|---|---|
| Dehydratisiertes Natriumriboflavinphosphatsalz | 0,147 g |
| Dextran T500 | 15 g |
| Acridingelb | 0,050 g |
| Dehydratisiertes monobasisches Natriumphosphat | 0,067 g |
| Dehydratisiertes zweibasisches Natriumphosphat | 0,285 g |
| Destilliertes Wasser | bis zu 100 g. |

9. Ophthalmische Zusammensetzung zur Anwendung bei der Behandlung von Keratokonus mittels kornealer Iontophorese nach Anspruch 1,
**dadurch gekennzeichnet, dass** sie nachstehende Formulierung aufweist:

| | |
|---|---|
| Dehydratisiertes Natriumriboflavinphosphatsalz | 0,147 g |
| Dextran T500 | 15 g |
| Erythrosin B | 0,050 g |
| Dehydratisiertes monobasisches Natriumphosphat | 0,067 g |
| Dehydratisiertes zweibasisches Natriumphosphat | 0,285 g |
| Destilliertes Wasser | bis zu 100 g |

**10.** Ophthalmische Zusammensetzung zur Anwendung bei der Behandlung von Keratokonus mittels kornealer Iontophorese nach Anspruch 1,
**dadurch gekennzeichnet, dass** sie nachstehende Formulierung aufweist

| | |
|---|---|
| Dehydratisiertes Natriumriboflavinphosphatsalz | 0,147 g |
| Dextran T500 | 15 g |
| Methylenblau | 0,050 g |
| Dehydratisiertes monobasisches Natriumphosphat | 0,067 g |
| Dehydratisiertes zweibasisches Natriumphosphat | 0,285 g |
| Destilliertes Wasser | bis zu 100 g. |

**11.** Ophthalmische Zusammensetzung zur Anwendung bei der Behandlung von Keratokonus mittels kornealer Iontophorese nach Anspruch 1,
**dadurch gekennzeichnet, dass** sie nachstehende Formulierung aufweist:

| | |
|---|---|
| Dehydratisiertes Natriumriboflavinphosphatsalz | 0,147 g |
| Dextran T500 | 15 g |
| Natrium-EDTA | 0,1 g |
| Tromethamin | 0,05 g |
| Dehydratisiertes monobasisches Natriumphosphat | 0,067 g |
| Dehydratisiertes zweibasisches Natriumphosphat | 0,285 g |
| Destilliertes Wasser | bis zu 100 g. |

**12.** Ophthalmische Zusammensetzung zur Anwendung bei der Behandlung von Keratokonus mittels kornealer Iontophorese nach einem der Ansprüche 5 bis 11, wobei das dehydratisierte monobasische Natriumphosphat und/oder das dehydratisierte zweibasische Natriumphosphat, die das Puffersystem bilden, in variablen Mengen auftreten, dergestalt, dass am Ende ein pH-Wert der Lösung von 5,5 erreicht wird.

**13.** Ophthalmische Zusammensetzung zur Anwendung bei der Behandlung von Keratokonus mittels kornealer Iontophorese nach Anspruch 12, wobei das dehydratisierte monobasische Natriumphosphat und/oder das dehydratisierte zweibasische Natriumphosphat durch irgendein Puffersystem (wie Citrat, Acetat, Weinsäure) ersetzt sind, das zum Erreichen eines pH-Wertes für 5,5 von Nutzen ist.

**14.** Ophthalmische Zusammensetzung nach einem der Ansprüche 1 bis 13 zur Anwendung bei der Behandlung von Keratokonus, **dadurch gekennzeichnet, dass** diese die nachstehenden Schritte vorsieht:

a. Anordnen einer iontophoretischen Vorrichtung auf dem zu behandelnden Auge, wobei die Vorrichtung einen Speicher umfasst, der die ophthalmische Lösung enthält;
b. Anlegen eines Stroms an die ophthalmische Lösung auf dem zu behandelnden Auge;
c. Steuern der Lösungsbewegung mittels eines 0,5 bis 5 min lang angelegten Kathodenstroms mit einer Stärke von nicht höher als 2 mA, und bevorzugt 1 mA.
d. Unmittelbar nach der Beendigung des Stromanlegens 5 bis 30 Minuten langes Bestrahlen der Korneaoberfläche mit einem UV-Licht mit einer Energie von 3 bis 30 mW/cm$^2$, wodurch eine Vernetzung der kornealen Kollagenfasern erhalten wird.

**Revendications**

1. Composition ophtalmique pour l'utilisation dans le traitement de kératocône par ionophorèse cornéenne, **caractérisée en ce qu'**elle comprend :

   A. des agents de réticulation permettant un renforcement de la structure de la cornée atteinte de kératocône grâce à l'entrelacement et l'augmentation de liaisons (réticulation) entre les fibres du collagène de la cornée et ayant des propriétés de tampon et dont une valeur de pH initiale est comprise entre 5 et 6, dans laquelle l'agent de réticulation est la riboflavine, ou une solution de phosphate de riboflavine, ou un sel de sodium déshydraté de phosphate de riboflavine, ou un sel de sodium déshydraté de phosphate de riboflavine.H$_2$O, ou un sel de sodium déshydraté de phosphate de riboflavine.2H$_2$O, et
   B. un système de tampon en quantités variables de manière à atteindre la valeur de pH de solution finale de 5,5, et
   C. des substances qui favorisent le passage de la riboflavine ou d'autres substances de photosensibilisation et de photo-polymérisation à travers l'épithélium cornéen, en permettant l'absorption par le stroma cornéen lui-même, dénommés des bio-améliorateurs,
   et/ou
   D. des substances photosensibles et de photo-polymérisation qui peuvent être facilement absorbées par l'épithélium et qui, comme la riboflavine, peuvent également être activées par la lumière pour former une réticulation de la cornée, dénommés photo-améliorateurs.

2. Composition ophtalmique pour l'utilisation selon la revendication 1, dans laquelle la concentration de la solution de riboflavine est comprise entre 0,1 et 1,0% en poids, et de préférence la concentration de la solution de riboflavine est comprise entre 0,1 et 0,4% en poids.

3. Composition ophtalmique pour l'utilisation selon la revendication 1, dans laquelle le bio-améliorateur est choisi dans la liste comprenant : l'EDTA associé à la trométhamine, des sels d'EDTA acceptables du point de vue ophtalmologique associés à la trométhamine, le polysorbate 80, la trométhamine, l'azone, le chlorure de benzalkonium, le chlorure de cétylpyridinium, le chlorure de cétyltriméthylammonium, l'acide laurique, le menthol, le méthoxy-salicylate, le polyoxyéthylène, le glycolate de sodium, le glycodéoxycolate de sodium, le sulfate sodique de lauryle, le salicylate de sodium, le taurocholate de sodium, taurodéoxycholate de sodium.

4. Composition ophtalmique pour l'utilisation selon la revendication 1, dans laquelle le photo-améliorateur est choisi dans la liste comprenant : le jaune d'acridine, le jaune de quinoléine, le bleu de méthylène et l'érythrosine.

5. Composition ophtalmique pour l'utilisation dans le traitement de kératocône par ionophorèse cornéenne selon la revendication 1, **caractérisée en ce qu'**elle a la formulation suivante :

   | | |
   |---|---|
   | Phosphate de riboflavine | 0,146 g |
   | Dextran T500 | 20, 00 g |
   | NaH$_2$PO$_4$.2H$_2$O | 0,225 g |
   | Na$_2$HPO$_4$.2H$_2$O | 0,950 g |
   | NaCl | 0,116 g |
   | H$_2$O | jusqu'à 100 g. |

6. Composition ophtalmique pour l'utilisation dans le traitement de kératocône par ionophorèse cornéenne selon la revendication 1, **caractérisée en ce qu'**elle a la formulation suivante :

   | Sel de sodium déshydraté de phosphate de | |
   |---|---|
   | Riboflavine | 0,147 g |
   | Dextran T500 | 15,00 g |

(suite)

| | |
|---|---|
| EDTA sodique | 0,1 g |
| Trométhamine | 0,1 g |
| Phosphate de sodium monobasique déshydraté | 0,067 g |
| Phosphate de sodium dibasique déshydraté | 0,285 g |
| Eau distillée | jusqu'à 100 g. |

**7.** Composition ophtalmique pour l'utilisation dans le traitement de kératocône par ionophorèse cornéenne selon la revendication 1, **caractérisée en ce qu'**elle a la formulation suivante :

| Sel de sodium déshydraté de phosphate de | |
|---|---|
| riboflavine | 0,147 g |
| Dextran T500 | 15 g |
| Jaune de quinoléine | 0,050 g |
| Phosphate de sodium monobasique déshydraté | 0,067 g |
| Phosphate de sodium dibasique déshydraté | 0,285 g |
| Eau distillée | jusqu'à 100 g. |

**8.** Composition ophtalmique pour l'utilisation dans le traitement de kératocône par ionophorèse cornéenne selon la revendication 1, **caractérisée en ce qu'**elle a la formulation suivante :

| Sel de sodium déshydraté de phosphate de | |
|---|---|
| riboflavine | 0,147 g |
| Dextran T500 | 15 g |
| Jaune d'acridine | 0,050 g |
| Phosphate de sodium monobasique déshydraté | 0,067 g |
| Phosphate de sodium dibasique déshydraté | 0,285 g |
| Eau distillée | jusqu'à 100 g. |

**9.** Composition ophtalmique pour l'utilisation dans le traitement de kératocône par ionophorèse cornéenne selon la revendication 1, **caractérisée en ce qu'**elle a la formulation suivante :

| Sel de sodium déshydraté de phosphate de | |
|---|---|
| riboflavine | 0,147 g |
| Dextran T500 | 15 g |
| Erythrosine B | 0,050 g |
| Phosphate de sodium monobasique déshydraté | 0,067 g |
| Phosphate de sodium dibasique déshydraté | 0,285 g |
| Eau distillée | jusqu'à 100 g. |

**10.** Composition ophtalmique pour l'utilisation dans le traitement de kératocône par ionophorèse cornéenne selon la revendication 1, **caractérisée en ce qu'**elle a la formulation suivante :

| Sel de sodium déshydraté de phosphate de | |
|---|---|
| riboflavine | 0,147 g |
| Dextran T500 | 15 g |
| Bleu de méthylène | 0,050 g |
| Phosphate de sodium monobasique déshydraté | 0,067 g |

(suite)

| | |
|---|---|
| Phosphate de sodium dibasique déshydraté | 0,285 g |
| Eau distillée | jusqu'à 100 g. |

**11.** Composition ophtalmique pour l'utilisation dans le traitement de kératocône par ionophorèse cornéenne selon la revendication 1, **caractérisée en ce qu'**elle a la formulation suivante :

| | |
|---|---|
| Sel de sodium déshydraté de phosphate de riboflavine | 0,147 g |
| Dextran T500 | 15 g |
| EDTA sodique | 0,1 g |
| Trométhamine | 0,05 g |
| Phosphate de sodium monobasique déshydraté | 0,067 g |
| Phosphate de sodium dibasique déshydraté | 0,285 g |
| Eau distillée | jusqu'à 100 g. |

**12.** Composition ophtalmique pour l'utilisation dans le traitement de kératocône par ionophorèse cornéenne selon l'une quelconque des revendications 5 à 11, dans laquelle le phosphate de sodium monobasique déshydraté et/ou le phosphate de sodium dibasique déshydraté, constituant le système de tampon, sont en quantités variables de manière à atteindre la valeur de pH de solution finale de 5,5.

**13.** Composition ophtalmique pour l'utilisation dans le traitement de kératocône par ionophorèse cornéenne selon la revendication 12, dans laquelle le phosphate de sodium monobasique déshydraté et/ou le phosphate de sodium dibasique déshydraté sont remplacés par n'importe quel système de tampon (tel que citrate, acétate, acide tartrique) utile pour atteindre une valeur de pH de 5,5.

**14.** Composition ophtalmique selon l'une quelconque des revendications 1 à 13 pour l'utilisation dans le traitement de kératocône, **caractérisée en ce qu'**elle prévoit les étapes suivantes :

a. le positionnement d'un dispositif ionophorétique sur l'oeil à traiter, le dispositif comprenant un réservoir contenant ladite solution ophtalmique ;
b. l'application d'un courant à ladite solution ophtalmique sur l'oeil à traiter ;
c. le pilotage du mouvement de solution par un courant cathodique appliqué pendant 0,5 à 5 minutes, à une intensité non supérieure à 2 mA, et de préférence 1 mA ;
d. l'irradiation, immédiatement après la fin de l'application de courant, de la surface de la cornée avec une lumière UV pendant 5 à 30 minutes à une puissance de 3 à 30 mW/cm$^2$ ; en obtenant ainsi la réticulation des fibres de collagène de la cornée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7331350 B
- US 20080015660 A
- WO 2009001396 A
- EP 20253321 A
- WO 2009073600 A
- IT 2009000392 W
- IT 200800472 A

**Non-patent literature cited in the description**

- **PRAUSNITZ M.R. ; NOONAN J.S.** Permeability of Cornea, Sclera, and Conjunctiva: A Literature Analysis for Drug Delivery to the Eye. *Journal of Pharmaceutical Sciences,* 1998, vol. 87, 1479-88
- **HUANG et al.** *Biophysical journal,* 1999